# EUROPÄISCHE PATENTANMELDUNG

(11) **EP 1 024 363 A2**
(43) Veröffentlichungstag der Anmeldung: **02.08.2000**
(21) Anmeldenummer: 00101102.2
(22) Anmeldetag: 20.01.2000
(51) Int. Cl.: G01N 33/53, G01N 33/543, C12Q 1/68, G01N 33/58, B01L 3/00

(54) **Quantitative Bestimmung von Analyten in einem heterogenen System**

(30) Priorität: 29.01.1999 DE 19903576
(71) Anmelder: BODENSEEWERK PERKIN-ELMER GMBH, D-88662 Überlingen (DE)
(72) Erfinder: Stemmler, Ivo, 72070 Tübingen (DE); Brecht, Andreas, 76337 Waldbronn (DE); Gauglitz, Günter, 72070 Tübingen (DE); Steinwand, Michael, 88696 Owingen (DE)
(74) Vertreter: Grünecker, Kinkeldey, Stockmair & Schwanhäusser Anwaltssozietät

(57) **Zusammenfassung**

Die Erfindung betrifft ein Verfahren zur quantitativen oder qualitativen Bestimmung eines Analyten oder dessen Interaktions- bzw. Reaktionskinetik in einem System mit wenigstens zwei verschiedene Phasen, umfassend den Schritt des Abnehmens wenigstens eines Meßsignals von mindestens einer der Phasen, wobei die verschiedenen Phasen bei der Abnahme des Meßsignals nebeneinander vorliegen und jedes Meßsignal einer der wenigstens zwei Phasen zugeordnet wird. Außerdem betrifft die Erfindung einen Probenträger, insbesondere zur Verwendung in dem erfindungsgemäßen Verfahren mit einer oder mehreren Kavitäten. Der Probenträger zeichnet sich dadurch aus, daß wenigstens ein Teil des Probenträgers wenigstens im Bereich einer oder mehrerer Kavitäten mit fluoreszenzlöschendem Material beschichtet ist.

## Beschreibung

Die vorliegende Erfindung betrifft ein Verfahren zum quantitativen Bestimmen eines Analyten in einem System, das mindestens zwei verschiedene Phasen umfaßt. Außerdem betrifft die vorliegende Erfindung einen Probenträger, der insbesondere zur Durchführung des erfindungsgemäßen Verfahrens geeignet ist.

Der Stand der Technik beschreibt zahlreiche Verfahren zur quantitativen Bestimmung eines Analyten in einer vorgegebenen Analysenprobe. Den einzelnen Nachweisreaktionen liegen verschiedene Prinzipien zugrunde. Hierzu zählen die Umsetzung des nachzuweisenden Analyten zu einer nachweisbaren Substanz, wobei z.B. eine farbige Verbindung erzeugt wird und der Grad der Farbbildung ein Maß für die Menge an vorliegendem Analyt ist. Andere Nachweisverfahren beruhen auf spezifischen Wechselwirkungen zwischen Analyt und einem Bindungspartner. Hierzu zählen beispielsweise die Nachweisverfahren unter Ausnutzung der spezifischen Wechselwirkung zwischen einem Antigen und einem Antikörper, einem Liganden und seinem dazugehörigen Rezeptor oder die Hybridisierung zwischen komplementären Nukleinsäuremolekülen. Diese Art Nachweisverfahren oder der Assays werden in der Regel auch als Affinitätsassays beschrieben. Bei den zugrunde liegende Affinitätsreaktionen kommt es in der Regel zu einer Bildung eines stöchiometrisch definierten, aber nicht kovalenten Komplexes aus dem für den Analyten spezifischen Bindungspartner (z.B. einem Rezeptor, Antikörper) und dem Analyten (z.B. einem Liganden, Antigen). Häufig sind Biomoleküle, wie Proteine, an diesen Reaktionen beteiligt. Es können jedoch auch Reaktionen zwischen niedermolekularen Substanzen, z.B. einem niedermolekularen Rezeptorliganden, und einer hochmolekularen Substanz, z.B. dem Rezeptor, vorliegen. Eine besondere Ausgestaltung dieser Affinitätsassays liegt dem Immunoassay zugrunde, bei dem die spezifische Interaktion zwischen einem Antikörper und einem Antigen ausgenutzt wird. Auf Nukleinsäureebene kann eine spezifische Interaktion zwischen zwei verschiedenen Nukleinsäuremolekülen über zueinander komplementäre Sequenzabschnitte erfolgen. Durch Hybridisierung der komplementären Sequenzen kommt es zur Ausbildung eines doppelsträngigen Nukleinsäuremoleküles.

Die genannten Assays finden ihre Anwendung auf zahlreichen technischen Gebieten. Hierzu zählen die klinische Analytik/Diagnostik, Umweltanalytik, Genomanalyse, Wirkstofftestung oder auch Genexpressionsstudien und Genbank-Screenings. Häufig werden mehrere hundert Proben parallel auf einem einzigen Probenträger untersucht. Diese sogenannte 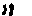 Mikroarray-Technik" erlangt heute zunehmend in sogenannten 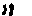 High Throughput Screenings" an Bedeutung.

Der Vorteil der auf Affinitätsreaktionen beruhenden Assays gegenüber auf chemischer Umsetzung des Analyten beruhenden Assays besteht darin, daß eine aufwendige Probenaufbereitung in der Regel nicht erforderlich ist. Vielmehr erfolgt die Abtrennung des Analyten von unerwünschten Verunreinigungen über die spezifische Wechselwirkung mit einem geeigneten Bindungspartner; der Bindungspartner fischt sozusagen den gewünschten Analyten gezielt aus der Analysenprobe heraus.

Eine besonders weit verbreitete Variante der Affinitätsassays stellen die Immunoassays dar, die auf der spezifischen Wechselwirkung zwischen Antikörper und Antigen beruhen. Bei dem sogenannten ELISA (Enzyme Linked Immuno Sorbent Assay) befindet sich einer der Reaktanden (d.h. entweder der Analyt oder der dazugehörige Bindungspartner) auf dem Probenträger, der häufig eine Mikrotiterplatte darstellt, in immobilisierter Form. Im Testverlauf bilden eine oder mehrere Komponenten des Testsystems einen Komplex mit der immobilisierten Komponente. Die Menge des gebildeten Komplexes dient als Maß für die Konzentration an Analyt in der Probe. Zwei gängige Varianten dieser Testformate bilden der sogenannte 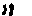 Sandwich-Assay" und der 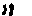 Kompetetive Assay". Bei den Sandwich-Formaten wird beispielsweise der Analyt durch zwei weitere Komponenten (oft zwei verschiedene Antikörper) komplexiert, so daß ein ternärer Komplex auf der Oberfläche des Probenträgers entsteht. Bei den kompetetiven Verfahren konkurrieren der Analyt und eine markierte Komponente, häufig der einen Marker tragende Analyt, um eine begrenzte Zahl von Bindungsstellen.

Ein Standardimmunoassay in heterogener Phase umfaßt häufig die folgenden Abläufe:
- Vorgeben eines festen Probenträgers;
- Zugeben von Analysenprobe und Nachweisreagenz;
- Abwarten des Einstellens des Bindungsgleichgewichts;
- Auswaschen ungebundener Anteile;
- Messen der gebundenen Anteile.

Gegebenenfalls werden diese Schritte bei komplexen Protokollen mehrfach wiederholt. Zum Schluß des gesamten Verfahrens schließt sich dann eine Detektion des gesamten Materials an, das im Verlauf des Verfahrens an den Probenträger gebunden wurde. Hierzu kann eine farbgebende Enzymreaktion, Elektrochemilumineszenz, Fluoreszenz, Radioaktivität usw. als Signalgeber eingesetzt werden.

Einige Assayformate arbeiten in homogener Phase, wie z.B. der Fluoreszenzpolarisationsimmunoassay FPIA, die aber in vielerlei Hinsicht komplex oder aber hinsichtlich der Ausgestaltung weniger flexibel als Assays in heterogener Phase sind.

Den genannten Assays ist praktisch allen gemeinsam, daß vor dem Abnehmen des Meßsignals eine Trennung von ungebundener Markierung (Aktivität, Meßsignal) und gebundener Markierung erfolgen muß. Dies wird in der Regel dadurch erreicht, daß der Probenträger vor der Messung (Abnahme des Meßsignals) einem oder mehreren Waschgängen unterzogen wird. Diese im Stand der Technik zwingend erforderlichen Waschvorgänge bringen jedoch Nachteile mit sich. Bei Probenträgern, die zahlreiche Nachweisreaktionen auf kleinem Raum gestatten, wie beispielsweise bei Mikrotiter- oder Nanotiterplatten, besteht das Problem des 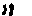 Übertrags", d.h. Probenaktivität wird durch das Waschen von einem Probenvolumen ins andere übertragen. Ein weiterer Nachteil der Verfahren, bei denen die physikalische Trennung von ungebundener und gebundener Markierung (oder Aktivität) erfolgt, besteht darin, daß keine zeitaufgelöste Beobachtung des Bindungsprozesses und mithin keine Untersuchung der Interaktions- oder Reaktionskinetik möglich ist.

Neben dem oben erwähnten Waschen zur Trennung von gebundener und ungebundener Aktivität erfolgt bei Verfahren unter Einsatz von Filterstreifen die Trennung zwischen ungebundener Aktivität und gebundener Aktivität durch Diffusion der Flüssigphase in der porösen Festphase, die durch den Filter gebildet wird. Diese Art Assays sind üblicherweise auf Einzelproben ausgelegt.

Der vorliegenden Erfindung lag das technische Problem zugrunde, ein Verfahren zur quantitativen oder qualitativen Bestimmung eines Analyten oder dessen Reaktions- oder Interaktionskinetik anzugeben, das die Nachteile aus dem Stand der Technik nicht mehr aufweist, insbesondere die im Stand der Technik erforderlichen Waschschritte vermeidet.

Ein weiteres Problem bestand darin, ein Verfahren der o.g. Art anzugeben, mit dem Probenvolumina von < 1 µl in Mikroarray-Anordnung analysiert werden können.

Ein weiteres Problem bestand auch in der Angabe von Mitteln, die zur Durchführung der erfindungsgemäßen Verfahren geeignet sind.

Das oben genannte technische Problem wird erfindungsgemäß gelöst durch ein Verfahren zur quantitativen oder qualitativen Bestimmung eines Analyten oder dessen Interaktions- bzw. Reaktionskinetik in einem System mit wenigstens zwei verschiedene Phasen, umfassend den Schritt des Abnehmens wenigstens eines Meßsignals von mindestens einer der Phasen, wobei die verschiedenen Phasen bei der Abnahme des Meßsignals nebeneinander vorliegen und jedes Meßsignal einer der wenigstens zwei Phasen zugeordnet wird.

Das genannte erfindungsgemäße Verfahren erlaubt die genannten Bestimmungen eines Analyten, ohne daß vor der Abnahme des wenigstens einen Meßsignals die physikalische Trennung zwischen ungebundener und gebundener Markierung erfolgen muß.

Das Verfahren ist zum qualitativen Nachweis des Vorliegens eines Analyten in einer Probe geeignet. Ferner können durch zeitlich aufeinanderfolgende Aufnahme von Meßsignalen beim Einstellen von Bindungsgleichgewichten bzw. dem Ablauf von chemischen Reaktionen die jeweiligen Kinetiken der Abläufe ermittelt werden. Vorzugsweise dient das Verfahren jedoch zur quantitativen Bestimmung eines Analyten in einer Analysenprobe.

Ein gängiger Verfahrensablauf läßt sich wie folgt beschreiben. Nach dem Einbringen der zu analysierenden Probe, beispielsweise in die Kavität einer Nanotiterplatte, die beispielsweise mit einem für den Analyten spezifischen Bindungspartner beschichtet ist, wird - im Falle eines kompetetiven Assays - eine definierte Menge des markierten Analyten zugesetzt. Nach Einstellen des Bindungsgleichgewichts wird das wenigstens eine Meßsignal von einem Volumenanteil der Flüssigphase abgenommen, wobei das von der Festphase erzeugte Signal im wesentlichen nicht erfaßt wird. Das von einer Phase - hier im Beispiel, der Flüssigphase - abgenommene Meßsignal dient dann zur Berechnung der in dieser Phase enthaltenen Menge an Analyt und letztendlich zur Bestimmung der in der untersuchten Proben vorhandenen Menge an Analyt. Die quantitative Auswertung des erhaltenen Meßsignals erfolgt mit einer vorher erstellen Eichkurve, bei der unter den gleichen Meßbedingungen die Signalstärke von vorgelegten, definierten Analytkonzentrationen ermittelt wurde.

Auch die qualitative Analyse ist auf diese Weise möglich. Wird ein Analyt beispielsweise im sogenannten Sandwich-Format nachgewiesen, kann z.B. ein zweiter markierter Antikörper nur an den ersten unmarkierten Antikörper der Festphase binden, wenn Analyt vorliegt. Der Nachweis eines Signals an der Festphase bedeutet somit, daß Analyt in der Probe vorliegen muß.

Schließlich lassen sich so Kinetiken ermitteln, in denen beispielsweise die Abnahme des Meßsignals in der Flüssigphase über den zeitlichen Verlauf hin verfolgt wird. Die zeitliche Änderung des Signals von der Flüssigphase ist des Maß für die untersuchte Kinetik einer Reaktion oder Interaktion des Analyten.

Das Ausmessen einer bestimmten Volumeneinheit einer der Phasen kann hierbei durch entsprechende Justierung der Meßvorrichtung sichergestellt werden. So läßt sich beispielsweise ein Laserstrahl, der zur Anregung fluoreszenzmarkierter Moleküle in der Analysenprobe eingesetzt werden kann, so ausrichten, daß lediglich ein bestimmter Volumenanteil einer Phase, z.B. der Flüssigphase, von dem Laserstrahl getroffen wird, und nur die in dem Strahlengang befindlichen Moleküle zur Fluoreszenz angeregt werden. Das so erhaltene Fluoreszenzsignal kann dann zur Berechnung der vorhandenen Analytkonzentration dienen.

Bei einer besonders bevorzugten Ausführungsform wird das Verfahren als Affinitätsassay durchgeführt. Hierbei wird die spezifische Wechselwirkung zwischen Analyt und einem Bindungspartner ausgenutzt. Beispiele für Analyt und dazugehöriger Bindungspartner sind Ligand als Analyt und Rezeptor als dazugehöriger Bindungspartner, Nukleinsäure als Analyt und komplementäre Nukleinsäure als Bindungspartner, Substrat als Analyt und dazugehöriges Enzym als Bindungspartner, Antigen als Analyt und Antikörper als entsprechender Bindungspartner. Eine ausführliche Übersicht über gängige Verfahren der Affinitätsanalyse findet sich in Price Ch. P, Newman D. J. (Herausgeber) 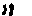 Principles and Practice of Immunoassay" Stockton Press (1991), New York.

Bei einer weiteren bevorzugten Ausführungsform wird das Verfahren als Immunaffinitätsassay durchgeführt. Bei dieser Art Assay wird für den Nachweis des Analyten die spezifische Wechselwirkung zwischen Antigen und Antikörper ausgenutzt. Als Antikörper für den spezifischen Nachweis des Analyten können sowohl monoklonale wie auch polyklonale Antikörper verwendet werden. Auch der Analyt, der als Antigen dient, kann seinerseits einen Antikörper darstellen.

Bei einer weiteren bevorzugten Ausführungsform beträgt das Volumen, in dem die Nachweisreaktion erfolgt, 1µl oder weniger, vorzugsweise beträgt das Volumen 50 bis 100 nl. Das Volumen der Nachweisreaktion entspricht dem eingesetzten Probenvolumen einschließlich der zugesetzten Reagenzien. Der Stand der Technik kennt keine Verfahren, bei denen derart kleine Probenvolumina einer der o.g. Bestimmung unterzogen werden können, insbesondere nicht, wenn sich die Proben in einem Mikroarray, wie einer Nanotiterplatte, befinden.

Bei einer weiteren bevorzugten Ausführungsform wird das Verfahren als kompetetiver Assay durchgeführt. Hierbei konkurrieren der nachzuweisende Analyt mit einer strukturell ähnlichen Verbindung, die in der Regel eine Markierung trägt, um eine begrenzte Anzahl an Bindungsstellen. Da die zugesetzte, markierte, zum Analyt kompetetive Substanz in konstanten Mengen zugesetzt wird, hängt die Anzahl von durch Analyt besetzten Bindungsstellen von der Analytkonzentration ab. Dies bedeutet, je mehr Analyt in der Probe vorliegt, um so weniger Bindungsstellen werden durch die markierte kompetetive Substanz besetzt. Dies wiederum bedeutet, daß bei zunehmender Analytkonzentation die Menge an ungebundenem, markiertem Kompetitor in der Flüssigphase zunimmt. Dies hat zur Folge, daß das von der Flüssigphase abgenommene Meßsignal zunimmt mit zunehmender Menge an Analyt in der Probe. Kompetetive Assays sind dem Fachmann aus dem Stand der Technik hinlänglich bekannt.

Das Verfahren kann jedoch alternativ als Sandwich-Assay durchgeführt werden. Hierbei wird beispielsweise der zu bestimmende Analyt mit zwei verschiedenen Antikörpern umgesetzt. Dabei ist ein erster Antikörper an die Festphase gebunden, und der zweite Antikörper trägt eine Markierung und wird der Analysenprobe zugesetzt. Nach beendeter Interaktion bildet sich ein ternärer Komplex an der Festphase der aus dem ersten Antikörper, dem Analyt und dem markierten zweiten Antikörper besteht. Dabei wird das Signal an der Festphase mit zunehmender Analytkonzentration zunehmen, während die Konzentration an markiertem zweitem Antikörper in der Flüssigphase mit zunehmender Analytkonzentration abnimmt. Wird bei der Detektion ein Volumenelement der Flüssigphase gemessen, so beobachtet man eine abnehmende Signalstärke mit zunehmender Analytkonzentration. Das Prinzip des Sandwich-Assays ist dem Fachmann aus dem Stand der Technik geläufig.

Das Meßsignal wird vorzugsweise durch eine Markierung erhalten, die Bestandteil des Analyten oder des Bindungspartners (Reaktants) vorliegt. Geeignete Markierungsmöglichkeiten sind dem Fachmann aus dem Stand der Technik bekannt. Hierzu zählen die radioaktive Markierung, eine durch Strahlungsanregung hervorgerufene Markierung, wie beispielsweise die Markierung mit Fluoreszenzmarkern, oder die Markierung mit einer Enzymaktivität. Als besonders bevorzugte Markierung wird eine fluoreszierende Gruppe in das jeweilige Molekül eingeführt. Dabei läßt sich die Fluoreszenz beispielsweise dadurch erzeugen, daß die markierten Moleküle mit einem Laserstrahl angeregt werden. Auch diese Technik ist dem Fachmann aus dem Stand der Technik hinlänglich bekannt.

Bei einer weiteren bevorzugten Ausführungsform umfaßt das System, mit dem das Verfahren durchgeführt wird, eine erste Phase, die eine Festphase darstellt, und eine zweite Phase, die eine Flüssigphase darstellt. Dabei trägt in der Regel die Festphase den spezifischen Bindungspartner für den nachzuweisenden Analyten, während die Flüssigphase durch die Probe, enthaltend den Analyt und die Nachweisreagenzien, gebildet wird. Bei einer weiteren Alternative kann die zweite Phase jedoch auch eine Gasphase sein, während die erste Phase die Festphase darstellt. Schließlich kann die Phasenkombination auch aus der Kombination einer Flüssigphase mit einer Gasphase bestehen.

Bei einer weiteren bevorzugten Ausführungsform wird die Festphase durch die Wandung einer Kavität in einem festen Probenträger gebildet. Dabei kann der feste Probenträger so ausgestattet sein, daß er nur zur Aufnahme einer einzelnen Probe geeignet ist. Der Probenträger kann aber auch so ausgebildet sein, daß er mehrere Proben gleichzeitig aufnehmen kann.

Bei einer besonders bevorzugten Ausführungsform ist der feste Probenträger eine Mikrotiterplatte, wie sie im Handel erhältlich ist. Besonders bevorzugt wird eine Nanotiterplatte als Probenträger verwendet, da diese eine große Anzahl an Kavitäten zur Probenaufnahme auf kleinem Raum aufweist. Die Kavitäten in dem festen Probenträger können verschiedene Formen aufweisen. Hierzu zählen die Quaderform oder Zylinderform, der Pyramidenstumpf und der Kegelstumpf. Besonders bevorzugt sind darüber hinaus Formen, deren Öffnungsfläche kleiner als deren Bodenfläche ist; hierzu zählen beispielsweise der negative Pyramidenstumpf und der negative Kegelstumpf. Bei dieser Ausführungsform wird die Beeinträchtigung des Meßergebnisses durch Streulicht/Fluoreszenz seitens der an den festen Probenträger gebundenen Markierung verringert im Vergleich zu entsprechenden Ausführungsformen, deren Bodenfläche kleiner als deren Öffnungsfläche ist (wie beispielsweise positiver Pyramidenstumpf, positiver Kegelstumpf).

Bei einer weiteren bevorzugten Ausführungsform wird der Einfluß von Störeffekten durch Markierung, die an die Phase gebunden ist, dadurch erniedrigt, daß die Phase eine quenchende Substanz enthält. Diese quenchende Substanz absorbiert das Signal, das von Molekülen erzeugt wird, die in unmittelbarer Nähe zu der quenchenden Substanz angeordnet sind. Vorzugsweise wird die quenchende Substanz so ausgewählt, daß sie die Fluoreszenz quencht, die erhalten wird, wenn die in dem System vorhandenen Moleküle durch einen Laser angeregt werden. Vorzugsweise quencht das Material die Fluoreszenz innerhalb einer kurzen Distanz, vorzugsweise von weniger als 100 nm. Als bevorzugte Materialien kommen Metalle, beispielsweise Gold oder Silber, aber auch Graphit oder Farbstoffe mit 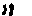 Quencheigenschaft" in Betracht.

Beispielsweise kann die Festphase eine derartige quenchende Substanz enthalten. Hierzu kann der Probenträgers mit quenchendern Material beschichtet werden. Dies ist insbesondere dann vorteilhaft, wenn nur die Fluoreszenz von in Lösung befindlichen Molekülen erfaßt werden soll.

Das Abnehmen des Meßsignals von nur einer der vorliegenden signalerzeugenden Phasen kann beispielsweise durch eine ortsaufgelöste Messung erhalten werden. Dies kann dadurch erfolgen, daß ein Laserstrahl die gesamte Kavität, in der sich die Probe befindet, abtastet und je nach Auflösungsvermögen mehrere Maßsignale erhalten werden. Die einzelnen Meßsignale repräsentieren die an dem jeweiligen Ort auftretende Fluoreszenzintensität und können somit zur Bestimmung der lokalen Konzentration an Analyt herangezogen werden.

Grundsätzlich reicht es allerdings aus, wenn nur ein einzelnes Meßsignal, das beispielsweise einem definierten Volumenelement der Flüssigphase entspricht, abgenommen wird.

Darüber hinaus kann durch Abnehmen mehrerer Meßsignale einer Phase, beispielsweise durch Abtasten der Probe mit einem Laserstrahl, und anschließendes Mitteln dieser Meßsignale die Statistik verbessert und somit die Bestimmung eines Analyten oder der Interaktions- bzw. Reaktionskinetik verbessert sowie der Fehler bei der Bestimmung eines Analyten oder der Interaktions- bzw. Reaktionskinetik verringert werden.

Gemäß einer weiteren bevorzugten Ausführungsform kann die quenchende Substanz so vorgesehen werden, daß die Strahlung einer Phase nahezu vollständig unterdrückt wird. Gemäß dieser Ausführungsform ist es nicht erforderlich, eine ortsaufgelöste Abnahme des wenigstens einen Meßsignals zur Zuordnung zur entsprechenden Phase durchzuführen. Werden beispielsweise die Wandungen und/oder der Boden der Kavität eines Probenträgers mit quenchendem Material beschichtet, kann demnach die Fluoreszenz, die von an der Wand gebundener Markierung resultiert, unterdrückt werden, und die Fluoreszenz, die von Markierung in der flüssigen Phase herrührt ohne das Erfordernis einer Ortsauflösung abgenommen werden.

Bei einer bevorzugten Ausführungsform wird in dem Probenvolumen ein Fleck mit einem Durchmesser von 40 µm oder weniger, vorzugsweise von rund 20 µm ausgeleuchtet und nur von diesem Volumenanteil das erzeugte Signal gemessen.

Das Ausleuchten des Probenvolumenanteils erfolgt vorzugsweise mit einem Laser, wobei das erzeugte Signal eine Fluoreszenz ist, die von den durch den Laser angeregten Fluarophor-tragenden Molekülen abgegeben wird.

Erfindungsgemäß wird außerdem ein Probenträger zur Verfügung gestellt, der eine oder mehrere Kavitäten aufweist und sich dadurch auszeichnet, daß wenigstens ein Teil des Probenträgers wenigstens im Bereich einer oder mehrerer Kavitäten mit fluoreszenzlöschendem Material beschichtet ist.

Dieser Probenträger kann in insbesondere in den oben beschriebenen Verfahren eingesetzt zur Unterdrückung der Fluoreszenzstrahlung einer Phase eingesetzt werden. Selbstverständlich sind die Einsatzmöglichkeiten dieses Probenträgers nicht auf die oben beschriebenen Verfahren beschränkt; vielmehr kann dieser Probenträger auch in anderen Verfahren eingesetzt werden, in denen es beispielsweise zur Verringerung von Streustrahlung erforderlich ist, Floureszenzstrahlung in einem bestimmten Bereich zu quenchen.

Gemäß einer vorteilhaften Ausführung kann das fluoreszenzlöschende Material ein Metall, wie beispielsweise Gold oder Silber umfassen. Falls erforderlich kann dieses Metall auch dotiert sein.

Die Kavität bzw. Kavitäten können vorteilhafterweise, entsprechend den Erfordernissen im Bereich des Bodens und/oder der Wandungen mit dem fluoreszenzlöschenden Material beschichtet sein.

Weiterhin können die bereits im Zusammenhang mit den Verfahren beschriebenen Vorteile durch verschiedene Formen der Kavitäten erzielt werden. So können im Probenträger Kavitäten vorgesehen sein, die eine Quaderform, eine Zylinderform, eine Pyramidenstumpfform oder eine Kegelstumpfform aufweisen. Es ist, wie ebenfalls oben beschrieben besonders vorteilhaft eine Kavität vorzusehen, deren Öffnungsfläche als deren Bodenfläche ist. Hierzu zählen insbesondere eine negative Pyramidenstumpfform oder eine negative Kegelstumpfform.

Gemäß einer weiteren vorteilhaften Ausführungsform ist der Probenträger in Form einer Mikrotiterplatte, vorzugsweise einer Nanotiterplatte, ausgebildet. Hierdurch ist es möglich eine Vielzahl von Proben zeitoptimiert zu analysieren.

Im folgenden werden bevorzugte Ausführungsformen der Erfindung und Beispiele des erfindungsgemäßen Verfahrens unter Bezugnahme auf die beigefügte Zeichnung beschrieben. In der Zeichnung zeigen:
- Fig. 1: eine schematische Darstellung einer Anordung zur Durchführung des erfindungsgemäßen Verfahrens,
- Fig. 2: eine Kavität eines Probenträgers gemäß einer Ausführungsform der vorliegenden Erfindung,
- Fig. 3: verschiedenen Formen von Kavitäten eines Probenträgers gemäß verschiedener Ausführungsformen der vorliegenden Erfindung,
- Fig. 4: eine Kalibrierkurve, welche die Fluoreszenzintensität in Abhängigkeit der Konzentration des Analyten (Atrazin) bei Verwendung eines monoklonalen Antikörpers gemäß eines ersten Beispiels des erfindungsgemäßen Verfahrens zeigt,
- Fig. 5: eine Kalibrierkurve, welche die Fluoreszenzintensität in Abhängigkeit der Konzentration des Analyten bei Verwendung eines polyklonalen Antikörpers gemäß eines zweiten Beispiels des erfindungsgemäßen Verfahrens
- Fig. 6: eine Darstellung zur Erläuterung des Zusammenhangs zwischen der Konzentration des Analyten und der Fluoreszenzintensität,
- Fig. 7: eine erste Darstellung zur Erläuterung der Bestimmung einer räumlichen Fluoreszenzverteilung gemäß einer weiteren Ausführungsform des erfindungsgemäßen Verfahrens, und
- Fig. 8: eine zweite Darstellung zur Erläuterung der Bestimmung einer räumlichen Fluoreszenzverteilung gemäß der weiteren Ausführungsform des erfindungsgemäßen Verfahrens.

### 1. Komponenten zur Durchführung des Verfahrens

### a) Probenträger

Es kann beispielsweise ein planarer Probenträger, der eine oder mehrere Kavitäten aufweist, die ein Flüssigkeitsvolumen von 1 µl oder weniger aufnehmen können, verwendet werden. Dessen Oberfläche wird mit einem Material belegt, das Fluoreszenz auf kurze Distanz (unter 100 nm) massiv reduziert (quencht). Das quenchende Material ist vorzugsweise ein Metall Gold und/oder Silber; es kann aber auch Graphit oder ein Farbstoff mit Quencheigenschaften sein. Die Oberfläche des Probenträgers wird weiterhin mit einem Bindungspartner für den Analyt belegt. Der Bindungspartner kann ein Antigen oder ein Antikörper sein. Der Bindungspartner kann entweder durch Adsorption oder durch kovalente Bindung an dem Probenträger fixiert werden. Geeignete Beschichtungsverfahren sind dem Fachmann bekannt.

### b) Reagenzien

Eine wesentliche Komponente stellt ein fluoreszenzmarkierter, analytspezifischer Bindungspartner (z.B. ein Antikörper) dar, der spezifisch an den Analyten binden kann. Grundsätzlich ist der Bindungspartner in der Lage, sowohl an freien Analyten in der Probe als auch an immobilisierten Analyt auf dem Probenträger zu binden. Verfahren zum Herstellen fluoreszenzmarkierter Antikörper sind dem Fachmann bekannt.

Als weitere Reagenzien kommen Substanzen zum Einsatz, die die unspezifische Bindung von Festbestandteilen, d.h. Analyt- oder Bindungspartner, an den Probenträger unterbinden oder reduzieren können. Zu diesem Zweck kommen beispielsweise Proteine oder Detergenzien in Betracht. Substanzen, die zum Unterdrücken unspezifischer Bindungen an Probenträger geeignet sind, sind dem Fachmann bekannt.

### c) Auslesevorrichtung

Es wird eine optische Vorrichtung verwendet, die es erlaubt, die in den Kavitäten vorliegenden fluoreszenzmarkierten Moleküle anzuregen und das emittierte Fluoreszenzlicht zu detektieren. Dabei kann die Fluoreszenz innerhalb jeder Kavität ortsaufgelöst angeregt und/oder ortsaufgelöst detektiert werden. Damit ist eine selektive Messung der in Lösung befindlichen Fluorophore möglich, ohne daß eine Trennung von gebundenen und freien Fluorophoren notwendig ist. Die selektive Messung kann durch verschiedene Ausgestaltungen der Meßvorrichtung erfolgen. Es ist möglich, die Anregungsoptik so auszugestalten, daß nur Fluorophore in Lösung angeregt werden, während Fluorophore an der Festphase nicht angeregt werden. Alternativ ist es möglich, durch Ausgestaltung der Detektionsoptik nur solche Fluoreszenz zu erfassen, die von Molekülen aus der Lösung emittiert wird, während Fluoreszenz, die von Festphase-gebundenen Molekülen emittiert wird, nicht erfaßt wird. Schließlich ist es möglich, durch die entsprechende Ausgestaltung des Probenträgers nur solche Fluoreszenz zu erfassen, die im wesentlichen von in Lösung befindlichen Molekülen stammt. Dies wird durch Kavitäten zur Probeaufnahme ermöglicht, die im Querschnitt eine negative Pyramidenstumpfform aufweisen, ermöglicht. Natürlich kann auch eine Kombination der genannten Alternativen den gewünschten Effekt erreichen.

### 2. Quantitative Bestimmung mit Hilfe eines kompetetiven Assays

Zur quantitativen Bestimmung eines gelösten Analyten in einer flüssigen Probe werden folgende Schritte durchgeführt:
- Es wird ein Probenträger bereitgestellt, bei dem eine oder mehrere Kavitäten mit einer Komponente des Testsystems, beispielsweise einem Antikörper, beschichtet sind. Diese Beschichtung kann zeitlich und räumlich von der Durchführung der eigentlichen quantitativen Bestimmung getrennt werden. Ferner kann der Probenträger in verschiedenen Kavitäten in unterschiedlicher Weise modifiziert sein.
- Die Probe und die erforderlichen Reagenzien werden in eine oder mehrere der Kavitäten gegeben. Gegebenenfalls kann die Kavität danach mit einem geeigneten Mittel zur weiteren Aufbewahrung versiegelt werden.
- Die Probe wird mit den Reagenzien inkubiert, bis sich die Bindungsgleichgewichte zwischen Analyt und Bindungspartner eingestellt haben.
- Die Fluoreszenz der in der Probe vorliegenden Moleküle wird unter Bedingungen angeregt bzw. detektiert, bei denen entweder im wesentlichen die in Lösung befindlichen Moleküle angeregt oder die an der Oberfläche gebundenen Moleküle angeregt werden. Möglich ist auch eine sequenzielle Anregung der auf die beiden Phasen verteilten Fluoreszenzmoleküle, etwa durch rasterförmige Abtastung des Probenträgers.
- Bei dem beschriebenen kompetetiven Assay wird das Fluoreszenzsignal in der Probenlösung mit der Analytkonzentration ansteigen, während das Fluoreszenzsignal an der Wandung des Probenträgers mit steigender Analytkonzentration abnehmen wird. Die Abnahme des Fluoreszenzsignals mit steigenden Analytkonzentrationen wird verstärkt, wenn die Oberfläche des Probenträgers mit einer Fluoreszenz-quenchenden Substanz beschichtet ist. Das erhaltene Signal kann in üblicher Weise durch Referenzmessungen kalibriert und mit der Konzentration des Analyten korreliert werden.

### 3. Meßvorrichtung zur Durchführung des Verfahrens

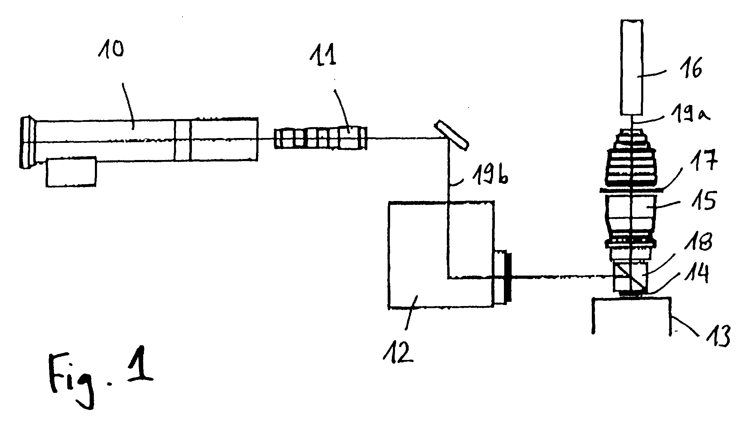

Fig. 1 zeigt schematisch eine mögliche Meßanordnung. Eine derartige Meßanordnung ist beispielsweise aus dem US-Patent 5,381,224 von Dixon bekannt. Diese Meßanordnung besteht aus einem Laser 10, einem serienmäßig lieferbaren Beamexpander 11, einer Abtasteinrichtung oder Scanner 12, einem speziell konstruierten Probentisch 13 zur Halterung eines Probenträgers 14, einer abbildenden Optik 15 sowie eine Detektionseinrichtung zum Nachweis der fluoreszierenden Strahlung 16, beispielsweise einem oder mehreren Photomultipliern. Ferner kann das System eine geeignete Filterkombination 17 aufweisen. Die Fluoreszenzanregung durch den Laser 10 erfolgt hierbei über den Beamexpander 11, den Scanner 13 und einen Strahlteiler 18, beispielsweise in Form eines dichroitischen Spiegels, auf der Probe. Die von der Probe emittierte Fluoreszenzstrahlung 19a wird in reflektierter Richtung durch den Strahlteiler 18 mit einer abbildenden Optik 15 in der Detektionseinrichtung 16 aufgefangen. Aus der Position des Laserstrahls 19b, die durch die kontrollierte Steuerung des Scanners 13 bekannt ist, kann jedes Lichtsignal eindeutig einem Punkt auf dem Probenträger 14 und damit einer zu vermessenden Probe bzw. Probenvolumen zugeordnet werden. Die Intensität des in der Detektionseinrichtung 16 erhaltenen Signals dient als quantitatives Maß für die Konzentration des Analyten in der Probe.

### 4. Ausgestaltung des Probenträgers

Der verwendete Probenträger besitzt mindestens eine Kavität, wird in der Regel aber mehr als eine Kavität umfassen. Dabei können sowohl Mikro- wie auch Nanotiterplatten als Probenträger dienen.

In Fig. 2 ist eine Teilansicht einer Mikrotiterplatte 20, die eine pyramidenstumpfförmige Kavität 21 zeigt, dargestellt. Die Mikrotiterplatte 20 ist mit einer fluoreszenzlöschende Schicht 22, beispielsweise aus Gold und/oder Silber, belegt. Die Beschichtung der Probenträger mit Gold als Quenchsubstanz kann mit einer Vakuumaufdampfanlage (Edwards 305) durch thermische Verdampfung von Gold durchgeführt werden. Die Probenträger werden zuerst mit Laborreiniger (Extran, Merk) gereinigt, getrocknet und dann in die Aufdampfkammer gegeben. Bei einem Vakuum von besser als 10⁻⁶ Bar haben sich Schichtdicken von 500 nm bis 1000 nm Gold als zweckmäßig erwiesen.

Gemäß Fig. 2 ist die gesamte Oberfläche der Mikrotiterplatte beschichtet. Dies führt zu einer Vereinfachung bei der Herstellung der Schicht. Eine vollständige Beschichtung der Mikrotiterplatte ist allerdings nicht erforderlich. Vielmehr können je nach den durch eine bestimmte Analyse vorgegebenen Bedingungen auch nur der Boden 23 und/oder die Wandungen 24 der Kavität beschichtet werden. Hergestellt wird der in Fig. 2 gezeigte Probenträger dadurch, daß durch die Form der Kavitäten durch verschiedene Naßätztechniken, beispielsweise anisotropes Naßätzen in ein Siliziumsubstrat 28 geätzt wird, und das Siliziumsubstrat anschließend mit einem Boden 29 versehen werden.

In Fig. 2 sind weiterhin an den Wandungen und dem Boden gebundenen Fluorophore 25 und in Lösung befindliche Fluorophore 26 dargestellt.

Von großer Bedeutung für die Qualität des erhaltenen Meßsignals ist die Ausgestaltung der Kavität. Durch die räumlich begrenzte Anregung mit einer fokussierten Laserlichtquelle werden überwiegend in Lösung befindliche Moleküle detektiert. Zur Verringerung der Fluoreszenzanregung der an die Festphase gebundenen Moleküle können senkrechte Wandungen 32 in den Kavitäten 31 verwendet werden, wie sie in der linken Abbildung von Fig. 3 gezeigt sind. Derartige senkrechte Wandungen finden sich in einer quaderförmigen oder zylinderförmigen Kavität.

Für eine nahezu vollständige Eliminierung der Fluoreszenzanregung der an den Wandungen gebundenen Moleküle sind insbesondere Formen einer Kavität geeignet, deren Öffnungsfläche 36 kleiner als deren Bodenfläche 37 ist. In der rechten Abbildung von Fig. 3 ist beispielsweise der Querschnitt einer derartigen Kavität 35 dargestellt. Diese Kavität 35 weist eine Pyramidenstumpfform oder eine Kegelstumpfform auf. Diese Ausführungsformen haben zur Folge, daß keine direkte Fluoreszenzanregung der an den Wandungen gebundenen Moleküle stattfindet und ferner auch keine Detektion einer Restfluoreszenz, initiiert durch Streulicht auf der wäßrigen Phase, erfolgt. Demzufolge wird bei Kavitäten dieser Form die Fluoreszenz von praktisch ausschließlich in der wäßrigen Phase befindlichen Molekülen gemessen.

### 5. Quantitative Bestimmung eines Pestizids

In dem Versuch wurde ein Pestizidderviat aus der Klasse der substituierten S-Triazine bestimmt. Als Rindungspartner für diesen Analyten wurden Antikörper eingesetzt.

Polyklonale Schafantikörper wurden aus Serum durch fraktionierte Ammonsulfatpräzipitation angereichert und über eine Affinitätssäule (Sephadex-Säule, enthaltend das Immunogen) isoliert.

Monoklonale Antikörper wurden aus Hybridomkulturüberstand (serumfreie Kultur) isoliert und über eine Protein-G-Säule gereinigt.

Zur Fluoreszenzmarkierung wurde ein kommerziell erhältlicher reaktiver Fluoreszenzfarbstoff (CY5-N-Hydroxysuccinimid, Amersham-Pharmacia-Biotech) eingesetzt. Die Markierung der Antikörper erfolgte nach Herstellerprotokoll. Die markierten Antikörper wurde durch Spindialyse (Amicon Konzentratoren, Aminco) gereinigt. Die Markierungsgrade wurden spektrophotometrisch bestimmt.

Konjugate von Rinderserumalbumin (RSA) und Hapten wurden wie folgt hergestellt. Aus einem Triazincarboxylderivat (Atrazin-Capronsäure) wurde in DMF mit Diisopropylcarbodiimid (Sigma) und N-Hydroxysuccinimid (Sigma) ein Aktivester hergestellt. 1 mg RSA in 100 mM Carbonatpuffer, pH 9,0, wurde mit einem Überschuß an Aktivester versetzt und für eine Stunde bei Raumtemperatur inkubiert. Das Konjugat wurde durch Spindialyse (Amicon Konzentrator, Aminco) gereinigt. Die Markierungsgrade wurden spektrophotometrisch bestimmt.

Die Beschichtung der Probenträger mit Gold als Quenchsubstanz erfolgte in einer Vakuumaufdampfanlage (Edwards 305) durch thermische Verdampfung von Gold. Die Probenträger wurden mit Laborreiniger (Extran, Merk) gereinigt, getrocknet und in die Aufdampfkammer gegeben. Bei einem Vakuum von besser als 10⁻⁶ Bar wurden 500 nm bis 1000 nm Gold aufgedampft. Zu Vergleichszwecken wurde jeweils ein Teil des Probenträgers beim Aufdampfvorgang abgedeckt. Nach dem Aufdampfen wurden die Goldschichten für einen Tag in eine Lösung von 0,2 % ω-Mercaptopropionsäure in Ethanol eingelegt, mit Ethanol gewaschen und getrocknet.

Die Oberflächen der Probenträger, z.B. der Mikrotiterplatten (Greiner Labortechnik) oder der Nanotiterplatten (GeSIM, Volumen der Näpfchen 600 µm x 600 µm bei 400 µm Tiefe, entspricht ca. 50 nl Volumen, pyramidenstumpfförmide Kavitäten, anisotrop geätzt in Silicium), wurden wie folgt mit dem Konjugat belegt. Die Oberfläche wurde für eine Stunde mit einer Lösung des Konjugats in phosphatgepufferter Saline, pH 7,4, inkubiert. Anschließend wurde die Oberfläche gewaschen und für eine weitere Stunde mit einer Lösung von 1 mg/ml RSA inkubiert, um unspezifische Bindungsstellen abzusättigen. Zur Stabilisierung der Schichten wurde für eine Stunde mit einer Lösung von 0,5 % Glutaranhydrid (Sigma) inkubiert. Anschließend wurden die Probenträger gewaschen und entweder sofort verwendet oder getrocknet und bei 4°C gelagert. Für die Vergleichsversuche wurden die Oberflächen nur mit RSA, nicht aber mit einem RSA-Pestizidkonjugat beschichtet.

### 5.1 Quantitative Bestimmung eines Atrazinderivats als Hapten bei mechanischer Separation von gebundenen und freien Antikörpern

In dem Versuch wurden die oben beschriebenen Atrazin-beschichteten Mikrotiterplatten eingesetzt. Der Versuch beschreibt die quantitative Bestimmung von Antikörpern, die gegen Atrazin gerichtet sind, wobei die Antikörper in der gelösten Fraktion nach Abtrennung von der Festphase bestimmt wurden.

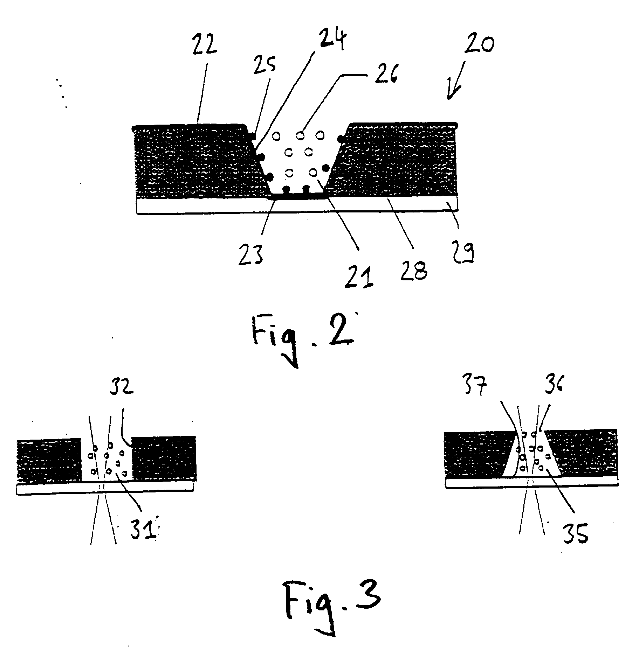

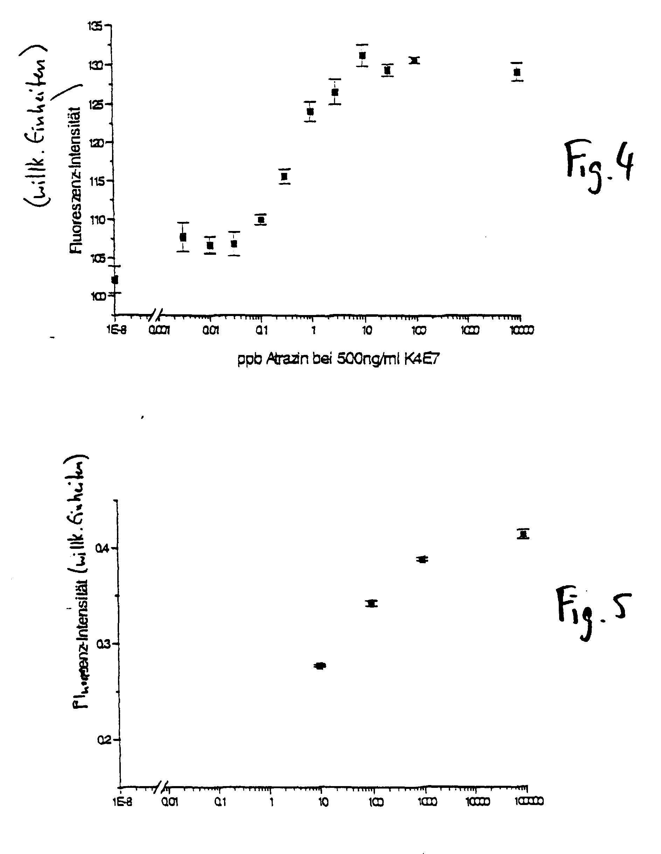

In jede Kavität einer Mikrotiterplatte mit einem Atrazinderivat, wie oben beschrieben, wurde zunächst 100 µl einer Atrazinlösung gegeben, gefolgt von 100 µl einer Lösung von fluoreszenzmarkierten anti-Atrazin-Antikörpern. Die Endkonzentration an Atrazin in der Lösung variierte von 0,003 µg/l bis 1000 µg/l. Die Antikörperkonzentration betrug jeweils 500 ng/ml. Nach einer Inkubationszeit von einer Stunde bei Raumtemperatur wurden aus jeder Kavität der Mikrotiterplatte 150 µl Lösung entnommen und in die Kavität einer opaque-weißen Fluoreszenzmikrotiterplatte (Perkin Elmer) gegeben. Die Fluoreszenz wurde mit einem Mikrotiterplatten-Fluoreszenzphotometer (Perkin Elmer LSR 200, Anregung bei 670 nm, Detektion bei 700 nm) vermessen. Fig. 4 zeigt die erhaltene Kalibrierkurve für einen monoklonalen Antikörper; Fig. 5 zeigt die Kalibrierkurve für einen polyklonalen Antikörper. Die Fluoreszenzintensitäten sind in willkürlichen Einheiten angegeben. Beide Kalibrierkurven zeigen einen eindeutigen und signifikanten Zusammenhang zwischen der Fluoreszenzintesität und der Konzentration des Analyten (Atrazin).

Sowohl im Falle der Verwendung von monoklonalen Antikörpern wie auch unter Verwendung der polyklonalen Antikörper war eine quantitative Bestimmung des Atrazins in einer Konzentration von weniger als 1 µg/l möglich.

### 5.2 Bestimmung der räumlichen Fluoreszenzverteilung auf einem miniaturisierten Probenträger

In diesem Versuch wird nachgewiesen, daß eine Anlagerung fluoreszenzmarkierter Bindemoleküle an die Wandungen des Probenträgers zu einer räumlichen Verteilung des Fluoreszenzsignals führt, die von der räumlichen Verteilung ohne Anlagerung der fluoreszenzmarkierten Bindemoleküle an die Wandung in eindeutiger und meßbarer Weise abweicht.

Ein GeSIM-Probenträger wurde, wie oben beschrieben, behandelt und auf einer Hälfte nur mit RSA, auf der anderen Hälfte mit RSA-Atrazinkonjugat belegt. In jede Kavität wurden mit einem Piezo-Microdropsystem (MICRODROP) 50 nl einer Losung von fluoreszenzmarkiertem anti-Atrazinantikörper in phosphatgepufferter Saline (pH 7.4) mit 100 µg/ml Ovalbumin gegeben. Die Konzentrationen an Antikörper waren 0.2 µg/ml 0.5 1 µg/ml und 1.0 µg/ml. Anschließend wurde die Platte mit einem transparenten Klebeband verschlossen (Adhesive Researeh) und für 30 Minuten bei Raumtemperatur inkubiert. Anschließend wurde mit einem Laser-scanner durch zeilenweise Abtastung des Probenträgers (Anregung durch das Klebeband bei 632 nm, Detektion mit einem Photomultiplier bei 690 nm) ein räumliches Abbild der Fluoreszenzintensität der Platte erzeugt. Im verwendeten Aufbau wurde auf dem Probenträger durch den Laser ein Fleck mit einem Durchmesser von ca. 20 µm ausgeleuchtet. Durch ein computergestütztes Datenerfassungssystem wurde ein Bild der Fluoreszenzintensitätsverteilung mit einer räumlicher Auflösung von 50 µm in willkürlichen Einheiten aufgezeichnet. Für jede Kavität ergab sich damit ein Bild von ca. 10 x 10 Bildpunkten.

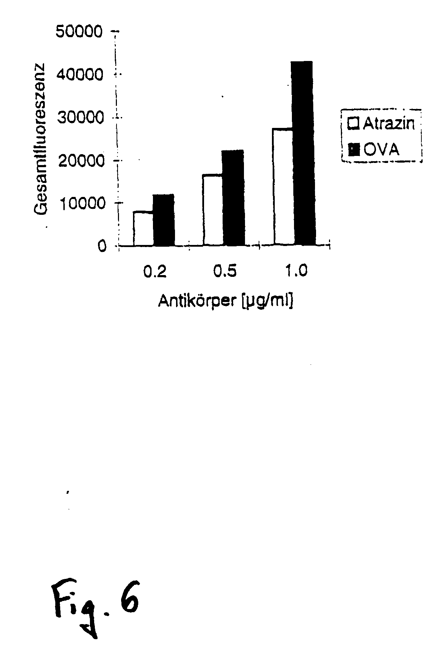

Zur Auswertung wurde die Fluoreszenzintensität für jede quadratische Kavität durch Summation über alle 100 zugeordneten Bildpunkte bestimmt. Das Ergebnis dieser Summation ist in Fig. 6 dargestellt. In Fig. 6 bezeichnen "Atrazin" die Signale aus Kavitäten, die mit einem Atrazin-Protein-Konjugat beschichtet worden sind. "OVA" bezeichnen die Kavitäten, die ausschließlich mit Ovalbumin beschichtet worden sind. Sowohl die Gesamtfluoreszenz für die mit einem AtrazinProtein-Konjugat beschichteten als auch die ausschließlich mit Ovalbumin beschichteten Kavitäten steigt mit der Konzentration des Antikörpers an.

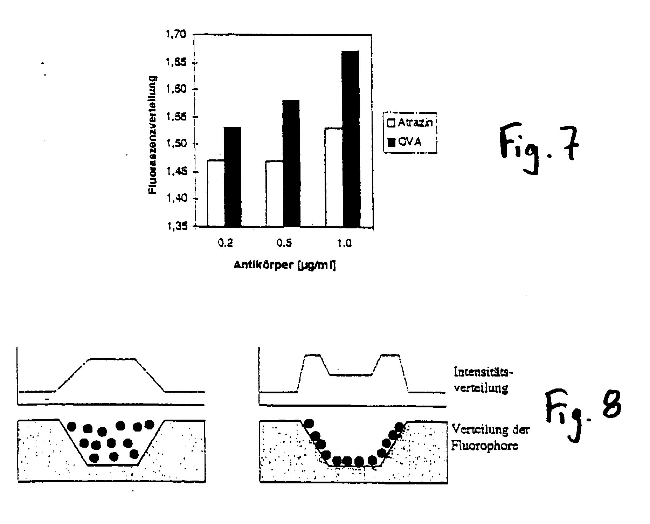

Um die räumliche Verteilung der Fluoreszenz zu bestimmen wurde die mittlere Intensität (I₄) pro Bildpunkt für ein Quadrat von 4 x 4 Bildpunkten in der Mitte jede Kavität, die mittlere Intensität (I₁₀) für die gesamte Kavität (10 x 10 Bildpunkte) bestimmt. Aus diesen beiden Werten wurde der Quotient I₄/I₁₀ bestimmt. Diese Ergebnisse sind in Fig. 7 dargestellt.

In Fig. 7 bezeichnen "Atrazin" wiederum die Signale aus Kavitäten, die mit einem Atrazin-Protein-Konjugat beschichtet worden sind, und "OVA" die Kavitäten, die ausschließlich mit Ovalbumin beschichtet worden sind. Für Kavitäten, in denen keine Bindung des Antikörpers an der Wandung stattfindet (OVA; siehe linke Abbildung in Fig. 8) ist die Mittenfluoreszenz im Vergleich zu den Kavitäten, bei denen der Antikörper an der Wandung bindet (Atrazin; siehe rechte Abbildung in Fig. 8) erhöht. Dieser Effekt ist weitgehend unabhängig von der Konzentration.

Hierbei ist zu beachten, daß der Laserstrahl durch die konkave Oberfläche des Flüssigkeitsmeniskus beim Eintritt in die flüssige Probe aufgeweitet wird. Dadurch und durch Reflexion in der Kavität ist die Ortsselektivität der Anregung begrenzt. Durch die angegebene erfindungsgemäße Ausgestaltung des Probenträgers, des Anregungssystems und/oder des Detektionssystems, kann daher eine deutlich bessere Signalcharakteristik erreicht werden. Für alle eingesetzten Konzentrationen an Antikörpern wird, wie erwartet, eine Abnahme der Fluoreszenzasymmetrie, d.h. eine geringere Betonung der Mitte der Vertiefung bei Bindung der Antikörper an die Wandung gefunden.

### 5.3 Quenching des Fluoreszenzsignals bei Anlagerung fluoreszenzmarkierter Bindemoleküle an eine mit fluoreszenzlöschendem Material beschichtete Wandung eines miniaturisierten Probenträgers

Der Versuch dient zum Nachweis, daß die Anlagerung fluoreszenzmarkierter Bindemoleküle an die goldbeschichteten Wandungen des Probenträgers zu einer signifikanten Abnahme des Fluoreszenzsignals führen, die durch Zugabe eines Analyten in der flüssigen Phase unterbunden werden kann, was das Verfahren für quantitative Bestimmungen geeignet macht.

Ein GeSIM-Probenträger wurde mit Gold bedampft, wie oben beschrieben, weiterbehandelt und auf einer Hälfte nur mit RSA, auf der anderen Hälfte mit RSA-Atrazinkonjugat belegt. In jede Kavität wurden mit einem Piezo-Microdropsystem (MICRODROP) 50nl einer Lösung von fluoreszenzmarkiertem anti-Atrazinantikörper 1 µg/ml) in phosphatgepufferter Sahne (pH 7.4) mit 100 µg/ml Ovalbumin gegeben. In einen Teil der Kavitäten wurde zusätzlich ein Atrazinderivat mit einer Konzentration von 100 ng/ml gegeben. Die folgende Tabelle gibt die gemittelten Wette für jeweils 10 Kavitäten an.

| | Fluoreszenz (willk. Einheiten) | % Fehler |
|---|---|---|
| 1 mg/ml Ovalbumin/PBS (Hintergrund) | 5149 | 1.7 |
| 1 µg/ml fluoreszenzmarkierter Antikörper ohne Atrazinderivat | 43574 | 1.8 |
| 1 µg/ml fluoreszenzmarkierter Antikörper mit 100 ng/ml Atrazinderivat | 65866 | 1.2 |

Die Fluoreszenz des Antikörpers nimmt bei Bindung an die Wandung um ca. 33% ab ("ohne Atrazinderivat"). Die entsprechend höhere Fluoreszenz bei Zusatz des Atrazinderivats erklärt sich aus Blockierung der Antikörperbindungsstellen. Damit werden die Antikörper nicht nahe an der fluoreszenzlöschenden Wandung das Probenträgers gebunden. Bei Einsatz eines lediglich mit RSA beschichteten Probenträgers wurde kein vergleichbarer Effekt gefunden. Die Reproduzierbarkeit des Verfahrens ist befriedigend und erlaubt eine Quantifizierung der Fluoreszenzänderungen.

## Patentansprüche

1. Verfahren zur quantitativen oder qualitativen Bestimmung eines Analyten oder dessen Interaktions- bzw. Reaktionskinetik in einem System mit wenigstens zwei verschiedene Phasen, umfassend den Schritt des Abnehmens wenigstens eines Meßsignals von mindestens einer der Phasen, wobei die verschiedenen Phasen bei der Abnahme des Meßsignals nebeneinander vorliegen und jedes Meßsignal einer der wenigstens zwei Phasen zugeordnet wird.

2. Verfahren nach Anspruch 1, in welchem das Verfahren als Affinitätsassay durchgeführt wird.

3. Verfahren nach Anspruch 1 oder 2, in welchem der Analyt eine Nukleinsäure darstellt.

4. Verfahren nach einem der Ansprüche 1 bis 3, in welchem das Verfahren als Immunaffinitätsassay durchgeführt wird.

5. Verfahren nach einem der Ansprüche 1 bis 4, in welchem das Volumen, in dem die Nachweisreaktion erfolgt, weniger als 1 µl ist, vorzugsweise im Bereich von 50 bis 100nl liegt.

6. Verfahren nach einem der Ansprüche 1 bis 5, in welchem das Verfahren als kompetitiver Assay durchgeführt wird.

7. Verfahren nach einem der Ansprüche 1 bis 5, in welchem das Verfahren als Sandwichassay durchgeführt wird.

8. Verfahren nach einem der Ansprüche 1 bis 7, in welchem der Analyt oder der Reaktant eine Markierung trägt, durch die das Meßsignal erzeugt wird.

9. Verfahren nach Anspruch 8, in weichem das Meßsignal durch Strahlungsanregung der Markierung erzeugt wird.

10. Verfahren nach Anspruch 8 oder 9, in welchem als Markierung eine Fluoreszenzmarkierung vorgesehen wird.

11. Verfahren nach einem der Ansprüche 1 bis 10, in welchem eine erste Phase als Festphase und eine zweite Phase als Flüssigphase vorgesehen wird.

12. Verfahren nach einem der Ansprüche 1 bis 11, in welchem die Festphase durch die Wandung einer Kavität in einem Probenträger gebildet wird.

13. Verfahren nach Anspruch 12, in welchem der Probenträger in Form einer Mikrotiterplatte, vorzugsweise einer Nanotiterplatte, vorgesehen wird.

14. Verfahren nach Anspruch 12 oder 13, in welchem eine Kavität vorgesehen wird, die eine Quaderform, eine Zylinderform, eine Pyramidenstumpfform oder eine Kegelstumpfform aufweist.

15. Verfahren nach Anspruch 12 oder 13, in welchem eine Kavität vorgesehen wird, deren Öffnungsfläche kleiner als deren Bodenfläche ist.

16. Verfahren nach Anspruch 15, in welchem eine Kavität vorgesehen wird, die eine Pyramidenstumpfform oder eine Kegelstumpfform aufweist.

17. Verfahren nach einem der Ansprüche 1 bis 16, in welchem eine quenchende Substanz zur Unterdrückung von Meßsignalen von einer der wenigstens zwei Phasen an diese Phase gekoppelt wird.

18. Verfahren nach einem der Ansprüche 11 bis 17, in welchem eine Kavität vorgesehen wird, deren Wandung und/oder der Boden mit einer quenchende Substanz, vorzugsweise einer fluoreszenzlöschenden Substanz, beschichtet ist.

19. Verfahren nach einem der Ansprüche 1 bis 18, in weichem das wenigstens eine Meßsignal durch ortsaufgelöste Messung erhalten wird.

20. Verfahren nach einem der Ansprüche 9 bis 19, in welchem das den markierten Analyten oder den markierten Reaktanden enthaltende Probenvolumen mit einem Lichtstrahl zur Anregung der Markierung bestrahlt wird, und die Abregungsstrahlung der Markierung als Meßsignal abgenommen wird.

21. Verfahren nach Anspruch 20, in welchem der Anregungslichtstrahl zur im Probenvolumen einen Strahldurchmesser von < 40 µm, vorzugsweise von rund 20 µm, aufweist.

22. Verfahren nach Anspruch 20 oder 21, in welchem der Anregungslichtstrahl zur Abnahme einer Mehrzahl von Meßsignalen über die Probe geführt wird.

23. Verfahren nach einem der Ansprüche 20 bis 22, in welchem das Anregen mit einem Laser erfolgt und als Meßsignal eine Fluoreszenz der durch den Laserstrahl angeregten Markierung abgenommen wird.

24. Probenträger (20), insbesondere zur Verwendung in einem Verfahren nach einem der Ansprüche 1 bis 23, mit einer oder mehreren Kavitäten (21)
**dadurch gekennzeichnet**, daß
wenigstens ein Teil des Probenträgers (20) wenigstens im Bereich einer oder mehrerer Kavitäten (21) mit fluoreszenzlöschendem Material (22) beschichtet ist.

25. Probenträger nach Anspruch 24, in welchem das fluoreszenzlöschende Material ein Metall umfaßt.

26. Probenträger nach Anspruch 25, in welchem das Metall dotiert ist.

27. Probenträger nach Anspruch 25 oder 26, in welchem das Metall Gold und/oder Silber umfaßt.

28. Probenträger nach einem der Ansprüche 24 bis 27, in welchem der Bereich den Boden (23) und/oder die Wandungen (24) einer oder mehrerer Kavitäten (21) umfaßt.

29. Probenträger nach einem der Ansprüche 24 bis 28, in welchem eine oder mehrere Kavitäten eine Quaderform (31), eine Zylinderform (31), eine Pyramidenstumpfform (21, 35) oder eine Kegelstumpfform (21, 35) aufweisen.

30. Probenträger nach einem der Ansprüche 24 bis 29, in welchem die Kavität eine Öffnungsfläche (36) aufweist, die kleiner als die Bodenfläche (37) der Kavität ist.

31. Probenträger nach Anspruch 30, in welchem die Kavität eine Pyramidenstumpfform (35) oder eine Kegelstumpfform (35) aufweist.

32. Probenträger nach einem der Ansprüche 24 bis 31, in welchem der Probenträger in Form einer Mikrotiterplatte, vorzugsweise einer Nanotiterplatte, ausgebildet ist.
